(19) Europäisches Patentamt
European Patent Office
Office européen des brevets

(11) Veröffentlichungsnummer: **0 008 402**
**B1**

(12) **EUROPÄISCHE PATENTSCHRIFT**

(45) Veröffentlichungstag der Patentschrift: **02.06.82**

(51) Int. Cl.³: **C 07 D 207/333,** C 07 C 119/20

(21) Anmeldenummer: **79102836.8**

(22) Anmeldetag: **07.08.79**

# (54) 2-(o-Hydroxyphenyl)-pyrrol und Verfahren zu seiner Herstellung.

(30) Priorität: **12.08.78 DE 2835439**

(43) Veröffentlichungstag der Anmeldung: **05.03.80 Patentblatt 80/5**

(45) Bekanntmachung des Hinweises auf die Patenterteilung: **02.06.82 Patentblatt 82/22**

(84) Benannte Vertragsstaaten: **AT BE CH DE FR GB IT LU NL SE**

(56) Entgegenhaltungen: **CH-A-579 045**

(73) Patentinhaber: **BASF Aktiengesellschaft, Carl-Bosch-Strasse 38, D-6700 Ludwigshafen (DE)**

(72) Erfinder: **Koenig, Horst, Dr. Chem., Pariser Strasse 4, D-6700 Ludwigshafen (DE)**
Erfinder: **Franke, Albrecht, Dr. Chem., Mandelring 11, D-6706 Wachenheim (DE)**
Erfinder: **Frickel, Fritz-Frieder, Dr. Chem., Prager Strasse 27, D-6700 Ludwigshafen (DE)**
Erfinder: **Steglich, Wolfgang, Dr. Chem., Am Klostergarten 3, D-5300 Bonn (DE)**
Erfinder: **Engel, Norbert, Goethestrasse 12, D-5300 Bonn (DE)**

**EP 0 008 402 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

2-(o-Hydroxyphenyl)-pyrrol und Verfahren zu seiner Herstellung

Die Erfindung betrifft 2-(o-Hydroxyphenyl)-pyrrol der Formel 1

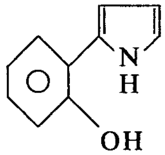

(1)

das ein wertvolles Zwischenprodukt für chemische Synthesen darstellt.

Das 2-(o-Hydroxyphenyl)-pyrrol wird hergestellt, indem man o-Benzyloxybenzoesäureallylamid in einem inerten Lösungsmittel mit Phosgen umsetzt und das erhaltene Imidchlorid in Gegenwart einer starken sterisch gehinderten Base cyclisiert und anschließend die Benzylschutzgruppe hydrogenolytisch abspaltet.

Das Herstellungsverfahren kann durch das folgende Formelschema veranschaulicht werden:

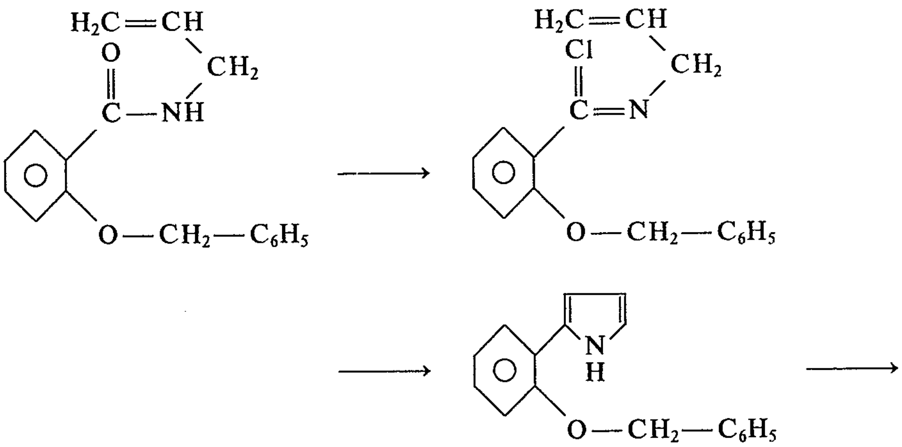

(1)

Die Ausgangsverbindung, das o-Benzyloxybenzoesäureallylamid, kann in an sich üblicher Weise aus o-Benzyloxybenzoesäuremethylester und Allylamin oder durch Umsetzung von O-Benzylsalicylsäurechlorid mit Allylamin erhalten werden.

Die Umsetzung von o-Benzyloxybenzoesäureallylamid mit Phosgen erfolgt durch Rühren zweckmäßig bei Raumtemperatur in einem inerten Lösungsmittel. Geeignete Lösungsmittel sind beispielsweise aromatische Kohlenwasserstoffe, insbesondere ein Benzolkohlenwasserstoff, wie Benzol, Toluol, halogenierte aliphatische Kohlenwasserstoffe, wie Methylenchlorid, Chloroform oder Tetrachlorkohlenstoff, Äther, insbesondere gesättigte cycloaliphatische Äther, wie Tetrahydrofuran oder Dioxan, oder Acetonitril.

In der Regel werden 20gewichtsprozentige Phosgenlösungen verwendet. In einer bevorzugten Ausführungsform wird dem Reaktionsgemisch eine geringe katalytische Menge Dimethylformamid zugesetzt.

Das bevorzugte Lösungsmittel für die Umsetzung mit Phosgen ist Toluol.

Anschließend wird, falls erforderlich, das verwendete Lösungsmittel zweckmäßigerweise bei erhöhten Temperaturen unter vermindertem Druck abdestilliert und das erhaltene Imidchlorid durch eine starke sterisch gehinderte Base cyclisiert.

Als starke sterisch gehinderte Basen kommen Natrium- oder Kaliumalkoholate des tert.-Butanols, des 2-Methylbutanol-2- oder beispielsweise das 1,5-Diazabicyclo 5, 4, 0 undecen-5 (DBU) in Betracht.

Die Cyclisierung wird vorteilhafterweise in einem ein- oder zweiwertigen gesättigten aliphatischen oder cyclischen Äther, wie Tetrahydrofuran, Dioxan, Glykoldimethyläther, oder in Dimethylformamid, Dimethylsulfoxid oder Hexamethylphosphorsäuretriamid oder einem Benzolkohlenwasserstoff, wie Benzol oder Toluol, als Lösungsmittel bei Temperaturen von 0 bis 150° C durchgeführt. Gegebenenfalls können auch Mischungen der genannten Lösungsmittel verwendet werden.

Es ist von Vorteil, die Cyclisierung unter Ausschluß von Luftsauerstoff unter Inertgas, beispielsweise unter Stickstoff oder Argon, durchzuführen.

Das bevorzugte Lösungsmittel für die Cyclisierungsreaktion ist Tetrahydrofuran oder eine Mischung aus Tetrahydrofuran und Benzol oder Toluol, und die bevorzugte Base ist Kalium-tert.-butylat.

Die Aufarbeitung des erhaltenen o-Benzyloxyphenyl-pyrrols erfolgt in üblicher Weise, wie Abdestillieren des Lösungsmittels, Verteilung in einem Zweiphasengemisch von Wasser und einem organischen Lösungsmittel, beispielsweise Methylenchlorid und Diäthyläther, und Abtrennung der

organischen Phase oder chromatographisch an einer Kieselgelsäule mit Methylenchlorid oder Tetrachlorkohlenstoff als Eluierungsmittel.

Die Benzylschutzgruppe wird in üblicher Weise durch katalytischen Wasserstoff in Gegenwart von Palladium-Kohle als Katalysator und in einem niederen Alkohol als Lösungsmittel, wie Methanol oder Äthanol, abgespalten.

Das erfindungsgemäße 2-(o-Hydroxyphenyl)-pyrrol ist ein wertvolles Zwischenprodukt für die Synthese pharmakologisch wirksamer Verbindungen. Wie beispielsweise aus der europäischen Patentanmeldung der Veröffentlichungsnummer 0 008 108 hervorgeht, können die Alkylamino-hydroxypropyläther der Formel (2)

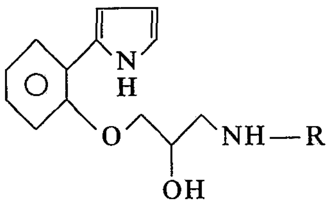

(2)

in der R einen am zum Stickstoffatom α-ständigen Kohlenstoffatom verzweigten Alkylrest mit 3 bis 6 Kohlenstoffatomen oder einen Alkinylrest mit 3 bis 6 C-Atomen oder Cyclopropyl bedeutet, durch Alkylierung von 2-(o-Hydroxyphenyl)-pyrrol mit einem Epihalogenhydrin oder einem α,ω-Dihalogen-2-propanol und anschließende Umsetzung mit einem Amin erhalten werden. Die Aminopropanol-Verbindungen der Formel (2) eignen sich aufgrund ihrer β-sympatholytischen Wirkung besonders zur Behandlung der coronaren Herzkrankheit, Herzrhythmusstörungen und der Hypertonie.

Beispiele für Verbindungen der Formel 2, die gegebenenfalls in Form eines ihrer physiologisch verträglichen Säureadditionssalze verwendet werden, sind:

2-[2-(3-tert.-Butylamino-2-hydroxypropoxy)-phenyl]-pyrrol
2-[2-(3-Isopropylamino-2-hydroxypropoxy)-phenyl]-pyrrol
2-[2-(3-Cyclopropylamino-2-hydroxypropoxy)-phenyl]-pyrrol
2-[2-(3-sec.-Butylamino-2-hydroxypropoxy)-phenyl]-pyrrol
2-[2-[3-(3-Methyl-1-butin-3-yl-amino)-2-hydroxypropoxy]-phenyl]-pyrrol
2-[2-[3-(1-Butin-3-yl-amino)-2-hydroxy-propoxy]-phenyl]-pyrrol.

Im Hinblick auf die Verwendung, Wirkung und Bestimmung von β-Blockern sei beispielsweise auf die Literaturstelle C. T. Dollery et al, Clinical Pharmacology and Therapeutics, Bd. 10, Nr. 6, 765—799, 1969, und den dort genannten Literaturstellen verwiesen.

Die Erfindung wird durch die nachfolgenden Ausführungsbeispiele näher erläutert.

I Synthese von 2-(o-Hydroxyphenyl)-pyrrol

a) o-Benzyloxybenzoesäureallylamid:

A) 30 g o-Benzyloxybenzoesäuremethylester und 15 ml Allylamin werden 7 Tage unter Rückfluß gekocht. Der Rückstand wird aus Ether bei −25° C umkristallisiert.
Ausbeute: 17,2 g, was 52% der Theorie entspricht; Schmp. 48° C.

B) 4,9 g (20 mmol) O-Benzylsalicylsäurechlorid in 10 ml Dioxan werden langsam in eine eisgekühlte Lösung von 1,7 g (30 mmol) Allylamin und 4 g Natriumhydrogencarbonat in 100 ml Wasser eingetragen und über Nacht gerührt. Das Produkt wird abgesaugt und wie unter A) umkristallisiert.
Ausbeute: 4,3 g, was 80% der Theorie entspricht.

b) 2-(o-Benzyloxyphenyl)-pyrrol:

2,7 g (10 mmol) o-Benzyloxybenzoesäureallylamid werden in 20 ml einer 20gewichtsprozentigen Lösung von Phosgen in Toluol unter Zusatz von 2 Tropfen Dimethylformamid über Nacht bei Raumtemperatur gerührt. Der nach dem Abdestillieren des Toluols unter vermindertem Druck bei 40° C Badtemperatur verbleibende Rückstand wird in 30 ml Tetrahydrofuran aufgenommen, über Glaswatte filtriert und innerhalb von 45 Minuten in eine eisgekühlte Lösung von 4,0 g (30 mmol) Kalium-tert.-butylat in 60 ml Tetrahydrofuran/Benzol (1 : 1 Vol.) unter Argon als Schutzgas eingetragen. 10 Minuten nach beendeter Zugabe wird das Lösungsmittel unter vermindertem Druck abdestilliert; man verteilt den Rückstand zwischen Methylenchlorid und Wasser und extrahiert

zweimal mit Methylenchlorid.

Die über Natriumsulfat getrockneten, vereinigten organischen Phasen werden eingedampft und der Rückstand über eine kurze Kieselgelsäure (30 × 2 cm) mit Tetrachlorkohlenstoff als Eluationsmittel chromatographiert. Das nach dem Einengen der Eluate verbleibende Öl wird aus Methanol umkristallisiert. Ausbeute: 1,4 g, das entspricht 56% der Theorie; Schmp. 72° C.

Analyse: $C_{17}H_{15}NO$ (249,3):

| | | | |
|---|---|---|---|
| ber. | 81,90 C | 6,06 H | 5,62 N |
| gef. | 81,76 C | 6,00 H | 5,45 N |

c) 2-(o-Hydroxyphenyl)-pyrrol:

Zu 1,5 g (6 mmol) 2-(o-Benzyloxyphenyl)pyrrol in 30 ml Methanol gibt man 150 mg 10%igen Palladium-Kohle-Katalysator und hydriert unter leichtem Überdruck. Der nach dem Abtrennen des Katalysators und Eindampfen verbleibende Rückstand wird aus Toluol/Petroläther (40°/60° C) umkristallisiert. Die Ausbeute von 0,8 g entspricht 84% der Theorie, Schmp. 100 bis 101° C.

Analyse: $C_{10}H_9NO$ (159,19):

| | | | |
|---|---|---|---|
| ber. | 75,45 C | 5,70 H | 8,80 N |
| gef. | 75,40 C | 5,70 H | 8,76 N |

II Synthese von 2-[2-(3-tert.-Butylamino-2-hydroxypropoxy)-phenyl]-pyrrol-fumarat

a) 2-[2-(2,3-Epoxypropoxy)-phenyl]-pyrrol

7,0 g 2-(o-Hydroxyphenyl)-pyrrol, 7,3 g Epibromhydrin und 11,4 g trockenes Kaliumcarbonat werden in 50 ml Aceton 7 Stunden auf Rückflußtemperatur erhitzt. Nach dem Erkalten wird abfiltriert und der Filterrückstand mit Aceton gewaschen. Die vereinigten Filtrate werden durch Abdestillieren vom Lösungsmittel befreit. Der zweimal an Kieselgel mit Methylenchlorid als Eluationsmittel chromatographierte Rückstand ergibt 4,6 g (48% d. Th.) 2-[2-(2,3-Epoxypropoxy)-phenyl]-pyrrol als farbloses Öl.

$^1H$-NMR-Spektrum ($CDCl_3$, TMS intern):

$\delta$ = 2,65 (m, 2H); 3,25—3,6 (m, 1H); 4,23 (m, 2H); 6,28 (m, 1H); 6,68 (m, 5H); 7,59 (m, 1H); 10,0 (breit, 1H)

b) 2-[2-(3-tert.-Butylamino-2-hydroxypropoxy)-phenyl]-pyrrol-fumarat

1,5 g 2-[2-(2,3-Epoxypropoxy)-phenyl]-pyrrol und 1 ml tert.-Butylamin werden in 5 ml Äthanol über Nacht belassen und anschließend das Lösungsmittel und überschüssiges Amin abdestilliert. Der wachsartige Rückstand wird in wenig Äthanol gelöst und tropfenweise mit ätherischer Fumarsäure versetzt. Das ausgefallene 2-[2-(3-tert.-Butylamino-2-hydroxypropoxy)-phenyl]-pyrrol-fumarat wird abgesaugt, mit trockenem Äther gewaschen und getrocknet.

Ausbeute: 1,8 g (74% der Theorie) vom Schmp. 197—198° C $C_{19}H_{26}O_4N_2$ (346)

| | | | |
|---|---|---|---|
| ber.: | 65,8 C | 7,6 H | 8,1 N |
| gef.: | 65,3 C | 7,5 H | 7,9 N |

Die Untersuchung der pharmakodynamischen Eigenschaften wurde nach folgenden Methoden vorgenommen.

Die $\beta$-sympatholytische Wirkung wurde im Vergleich mit dem bekannten $\beta$-Sympatholytikum Propranolol (1-(Isopropylamino)-1-naphthyloxy-2-propanol · HCl) an Ratten getestet.

1. $\beta_1$-sympatholytische Wirkung

Isoproterenol (0,1 ug/kg intravenös) verursacht an despinalisierten Ratten (Stamm: Strague-Dawley, Mus Rattus; Gewicht 230 bis 280 g) Herzfrequenzsteigerungen um durchschnittlich 45%. $\beta$-Sympatholytica hemmen diese Tachycardien. Isoproterenol wurde vor und 5 min nach der intravenösen Applikation der Prüfsubstanzen appliziert. Zwischen den Logarithmen der applizierten Dosen (mg/kg) der Prüfsubstanzen und der Hemmung der Isoproterenol-Tachycardie (%) bestehen lineare Beziehungen. Aus diesen Beziehungen werden als ED 50% die Dosen ermittelt, welche die Isoproterenol-Tachycardie um 50% hemmen.

2. Akute Toxizität

Die akute Toxizität wurde an Gruppen von je 10 weiblichen NMRI-Mäusen, Gewicht 19 bis 27 g, bei intraperitonealer Applikation ermittelt. Als LD 50 wurde die Dosis berechnet (Probit-Analyse), nach der 50% der Tiere innerhalb von 24 Stunden starben.

Die Tabelle 1 zeigt, daß die pharmakotherapeutisch wichtige $\beta_1$-sympatholytische Aktivität von 2-[2-(3-tert.-Butylamino-2-hydroxypropoxy)-phenyl]-pyrrol-fumarat 5,6mal stärker ist als die der Vergleichssubstanz Propranolol. Die therapeutische Breite als Quotient aus der letalen Dosis (LD 50) und der $\beta_1$-blockierenden Dosis (ED 50%) ist hierbei 7mal größer als die von Propranolol.

Tabelle 1

| Verbindung | $\beta_1$-sympatholytische Wirkung¹) | | Akute Tox. | Therapeutische Breite⁵) | |
|---|---|---|---|---|---|
| | ED 50%²) | R. W.³) | LD 50⁴) | absolut | relativ⁶) |
| Propranolol | 0,0127 | 1,00 | 108 | 111 | 1,00 |
| 2-[2-(3-tert.-Butylamino-2-hydroxy-propoxy)-phenyl]-pyrrol-fumarat | 0,00227 | 5,59 | 165 | 767 | 6,91 |

[1]) Hemmung der Isoproterenol-Tachycardie. Ratte despinalisiert. Appl.: i. V.
[2]) Dosis, welche die Isoproterenol-Tachycardie um 50% hemmt.
[3]) Relative Wirksamkeit. Propranolol = 1,00.
[4]) Maus. App.: i. p.
[5]) $\frac{\text{LD 50}}{\text{ED 50\%}}$
[6]) Propranolol = 1,00.

**Patentansprüche für die Vertragsstaaten BE, CH, DE, FR, GB, IT, LU, NL, SE.**

1. 2-(o-Hydroxyphenyl)-pyrrol.
2. Verfahren zur Herstellung von 2-(o-Hydroxyphenyl)-pyrrol, dadurch gekennzeichnet, daß man o-Benzyloxybenzoesäureallylamid in einem inerten Lösungsmittel mit Phosgen umsetzt, das erhaltene Imidchlorid in Gegenwart einer starken sterisch geminderten Base cyclisiert und anschließend die Benzylschutzgruppe hydrogenolytisch abspaltet.

**Patentanspruch für den Vertragsstaat AT**

Verfahren zur Herstellung von 2-(o-Hydroxyphenyl)-pyrrol, dadurch gekennzeichnet, daß man o-Benzyloxybenzoesäureallylamid in einem inerten Lösungsmittel mit Phosgen umsetzt, das erhaltene Imidchlorid in Gegenwart einer starken sterisch gehinderten Base cyclisiert und anschließend die Benzylschutzgruppe hydrogenolytisch abspaltet.

**Claims for the contracting states BE, CH, DE, FR, GB, IT, LU, NL, SE**

1. 2-(o-Hydroxyphenyl)-pyrrole.
2. A process for the preparation of 2-(o-hydroxyphenyl)-pyrrole, characterized in that o-benzyloxybenzoic acid allylamide is reacted with phosgene in an inert solvent, the resulting imide-chloride is cyclized in the presence of a strong sterically hindered base and the benzyl protective group is then removed by hydrogenolysis.

**Claim for the contracting state AT**

A process for the preparation of 2-(o-hydroxyphenyl)-pyrrole, characterized in that o-benzyloxybenzoic acid allylamide is reacted with phosgene in an inert solvent, the resulting imide-chloride is cyclized in the presence of a strong sterically hindered base and the benzyl protective group is then removed by hydrogenolysis.

**Revendications pour les États contractants BE, CH, DE, FR, GB, IT, LU, NL, SE**

1. 2-(o-hydroxyphényl)-pyrrole.

2. Procédé de préparation de 2-(o-hydroxyphényl)-pyrrole, caractérisé par le fait qu'on fait réagir de l'allylamide d'acide o-benzyloxybenzoique dans un solvant inerte avec du phosgène, on cyclise le chlorure d'imide obtenu, en présence d'une base forte, stériquement empêchée, et on sépare ensuite hydrogénolytiquement le groupe protecteur benzyle.

**Revendication pour l'État contractant AT**

Procédé de préparation de 2-(o-hydroxyphényl)-pyrrole, caractérisé par le fait qu'on fait réagir de l'allylamide d'acide o-benzyloxybenzoique dans un solvant inerte avec du phosgène, on cyclise le chlorure d'imide obtenu, en présence d'une base forte, stériquement empêchée, et on sépare ensuite hydrogénolytiquement le groupe protecteur benzyle.